# EUROPEAN PATENT SPECIFICATION

(11) **EP 3 372 252 B1**
(45) Date of publication and mention of the grant of the patent: **22.09.2021**
(21) Application number: 16861836.1
(22) Date of filing: 08.09.2016
(51) Int. Cl.: C02F 1/32, C02F 1/44, A61L 2/10, B01D 63/02

(54) **FLUID STERILIZATION APPARATUS**
FLUIDSTERILISATIONSVORRICHTUNG
APPAREIL DE STÉRILISATION DE FLUIDE

(30) Priority: 04.11.2015 JP 2015216983
(43) Date of publication of application: 12.09.2018
(73) Proprietor: Nikkiso Co., Ltd., Tokyo 150-6022 (JP)
(72) Inventor: OCHI, Tetsumi, Hakusan-shi Ishikawa 924-0004 (JP); CHIBA, Toshiaki, Tokyo 150-6022 (JP)
(74) Representative: Copsey, Timothy Graham
(86) International application number: PCT/JP2016/076423
(87) International publication number: WO 2017/077767

(56) References cited:
- EP-A1- 2 284 127
- CN-Y- 2 625 381
- JP-A- H11 290 656
- JP-A- H11 309 202
- JP-A- 2001 340 880
- JP-A- 2004 223 000
- JP-A- 2010 264 238
- JP-A- 2014 233 712
- JP-U- 3 045 089
- JP-U- H0 455 353
- JP-U- S6 095 997
- KR-A- 20120 074 535

## Description

### [TECHNICAL FIELD]

The present invention relates to fluid sterilization devices and, more particularly, to a technology of sterilizing a fluid by irradiating the fluid with ultraviolet light.

### [BACKGROUND ART]

It is known that ultraviolet light has sterilization capability. Devices that radiate ultraviolet light are used for sterilization in medical and food processing fronts. Devices that sterilize a fluid such as water continuously by irradiating the fluid with ultraviolet light are also used. One example is a device in which an ultraviolet LED is provided on the inner wall at a pipe end of a flow passage formed by a straight metal pipe (see JP2011-16074). Further examples of known devices for fluid sterilization are CN 2 625 381Y, KR 2012 0074535, JP H11 290 656 and EP 2 284 127.

### [PROBLEM TO BE SOLVED BY THE INVENTION]

In order to irradiate a fluid with ultraviolet light with a high efficiency, it is desirable to control the state of flow of the fluid in the flow passage appropriately and to radiate ultraviolet light in a mode suitable to the state of flow.

In this background, one illustrative purpose of the present invention is to provide a fluid sterilization device in which the cleaning capability is enhanced.

### [MEANS TO SOLVE THE PROBLEM]

The present invention is as defined in the appended claims.

A
fluid sterilization device according to an embodiment of the present invention comprises: a housing including a first housing that defines an upstream side of a flow passage extending in a longitudinal direction and a second housing that defines a downstream side of the flow passage and that is connectable to the first housing; a hollow fibre filter provided in the first housing and extending in the longitudinal direction; and a light source provided in the housing and including a light emitting device that emits ultraviolet light, a reflector that is provided to surround the light emitting diode and a sealing window that seals the light emitting diode and the reflector, where the light source is positioned near an exit of the hollow fibre filter on the central axis of the housing to radiate the ultraviolet light toward the downstream side of the flow passage opposite to the hollow fibre filter and wherein the reflector reflects the ultraviolet light travelling from the light emitting diode toward the filter in the direction of the downstream side of the flow passage so as to irradiate a fluid flowing in a laminar flow state in the second housing after passing through the hollow fibre filter with the ultraviolet light.

According to this embodiment, the fluid turned into a laminar flow by the filter extending in the longitudinal direction is irradiated with ultraviolet light so that the sterilization efficiency is increased as compared with the case of irradiating the fluid in a turbulent flow state with ultraviolet light. We have found that sterilization by ultraviolet irradiation in a straight flow passage is about 5-7 times more efficient in the case of a laminar flow state than in the case of a turbulent flow state. According to this embodiment, the filter removes fine particles etc. included in the fluid, and the sterilization effect from ultraviolet irradiation is enhanced by turning the fluid into a laminar flow by the filter. Accordingly, the cleaning capability of the fluid sterilization device is improved.

The filter may be a hollow fiber filter.

The light source may be provided to radiate ultraviolet light in the longitudinal direction.

The light source may be provided near an exit of the filter and radiate ultraviolet toward a downstream side of the flow passage.

The fluid sterilization device may further comprise a support structure for fixing the light source near a central axis of the flow passage.

The support structure may have a streamlined cross section along the longitudinal direction.

The light source may be provided at an end of the flow passage and radiate ultraviolet light toward an upstream side of the flow passage.

The fluid sterilization device may further comprise a straightener made of a material that reflects the ultraviolet light emitted by the light source and provided near an exit of the filter.

### [ADVANTAGE OF THE INVENTION]

According to the present invention, the efficiency of irradiating the fluid flowing in the flow passage with ultraviolet light is increased and the sterilization capability is improved.

### [BRIEF DESCRIPTION OF THE DRAWINGS]

Fig. 1 schematically shows a configuration of a fluid sterilization device according to the first embodiment;
Fig. 2 is a cross sectional view schematically showing a configuration of a fluid sterilization device according to the second embodiment;
Fig. 3 is a cross sectional view schematically showing a configuration of a fluid sterilization device according to the third embodiment;
Figs. 4A and 4B are cross sectional views schematically showing a configuration of the support structure;
Fig. 5 is a cross sectional view schematically showing a configuration of a fluid sterilization device according to the fourth embodiment; and
Fig. 6 is a cross sectional view schematically showing a configuration of a fluid sterilization device according to the fifth embodiment.

### [MODE FOR CARRYING OUT THE INVENTION]

A description will be given of the embodiments of the present invention with reference to the drawings. Like numerals are used in the description to denote like elements and the description is omitted as appropriate.

### (First embodiment)

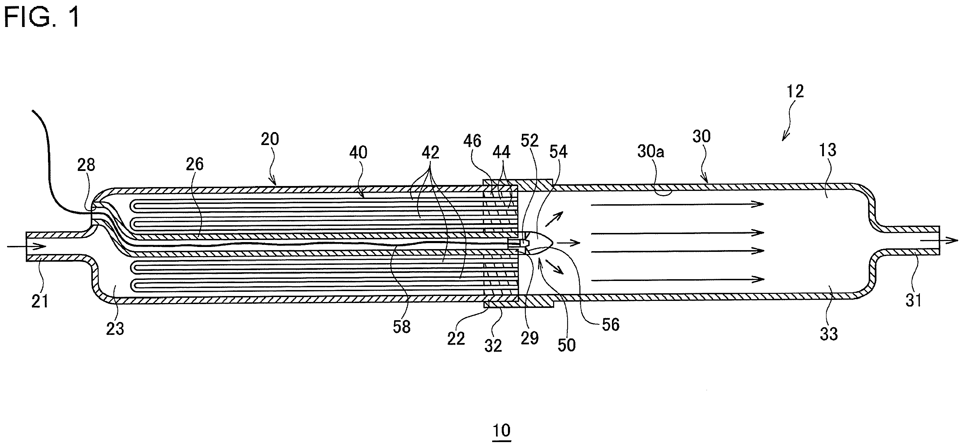

Fig. 1 schematically shows a configuration of a fluid sterilization device 10 according to the first embodiment. The fluid sterilization device 10 includes a housing 12 that defines a flow passage 13 extending in the longitudinal direction, a filter 40, and a light source 50. The fluid sterilization device 10 irradiates the fluid passing through the filter 40 and flowing in a laminar flow state with ultraviolet light from the light source 50 so as to sterilize the fluid such as water flowing in the flow passage 13.

The housing 12 includes a first housing 20 and a second housing 30. The first housing 20 includes an inflow end 21 and a first connection end 22 and is a straight pipe member extending from the inflow end 21 to the first connection end 22 in the longitudinal direction and defining a first flow passage 23. The second housing 30 includes an outflow end 31 and a second connection end 32 and is a straight pipe member extending from the second connection end 32 to the outflow end 31 in the longitudinal direction and defining a second flow passage 33. The outer circumference of the first connection end 22 and the inner circumference of the second connection end 32 are threaded (not shown). The first connection end 22 and the second connection end 32 are threadably engaged so as to connect the first housing 20 and the second housing 30.

The first housing 20 and the second housing 30 form the flow passage 13 extending from the inflow end 21 to the outflow end 31 in the longitudinal direction. The fluid flowing in the flow passage 13 flows in from the inflow end 21 in the axial direction and flows out from the outflow end 31 in the axial direction. In this specification, the direction along the flow passage 13 may be referred to as the longitudinal direction or the axial direction, and the direction perpendicular to the axial direction may be referred to as the radial direction. The position close to the inflow end 21 may be referred to as the upstream side and the position close to the outflow end 31 may be referred to as the downstream side to indicate the relative positions in the axial direction.

The first housing 20 includes a support structure 26. The support structure 26 is a pipe member extending in the longitudinal direction near the central axis of the first housing 20. An end 29 of the support structure 26 is positioned near the first connection end 22 and a light source 50 is mounted to the end 29. The support structure 26 is curved in the radial direction at a position near the inflow end 21 and is connected to a wiring hole 28 provided on the wall surface of the first housing 20. Inside the support structure 26 is provided a wiring 58 for supplying electric power to the light source 50.

The first housing 20 and the second housing 30 are made of a metal material or a resin material. The second housing 30 is desirably made of a material having a high ultraviolet reflectivity. For example, the second housing 30 is made of mirror-polished aluminum (Al) or polytetrafluoroethylene (PTFE), which is a fully fluorinated resin. In particular, by forming the inner wall surface 30a of the second housing 30 using a material having a high ultraviolet reflectivity, the ultraviolet light emitted by the light source 50 can be reflected by the inner wall surface 30a to irradiate the fluid flowing in the second flow passage 33 again. PTFE is a chemically stable material and has a high ultraviolet reflectivity and so is suitable as the material for the second housing 30. The first housing 20 may be made of aluminum or PTFE.

The filter 40 is provided inside the first housing 20. The filter 40 is a so-called hollow fiber filter and has a plurality of hollow fibers 42 extending in the axial direction of the first flow passage 23. The plurality of hollow fibers 42 are bent to half so that their ends 44 are aligned with the position of the first connection end 22 and are bundled. The ends 44 of the plurality of hollow fibers 42 are fixed by a sealant 46 that fills a space around the first connection end 22. The openings of the ends 44 are exposed at the downstream end face of the sealant 46. It is preferable that the plurality of hollow fibers 42 be provided to extend in the longitudinal direction. It is preferable that the plurality of hollow fibers 42 be provided to extend over the entirety of the length of the first flow passage 23 as illustrated.

The light source 50 includes a light emitting device 52, a sealing window 54 and a reflector 56. The light source 50 radiates ultraviolet light toward the fluid flowing in the second flow passage 33. The light source 50 is provided at the end 29 of the support structure 26 and is positioned such that its position in the radial direction is located on the central axis of the flow passage 13 or near the central axis. Further, the light source 50 is positioned such that its position in the axial direction is near the boundary between the first housing 20 and the second housing 30 or near the exit of the filter 40.

The light emitting device 52 is a light emitting diode (LEDs) configured to emit ultraviolet light, and the central wavelength or peak wavelength thereof is included in a range of about 200 nm - 350 nm. It is preferable that the light emitting device 52 emit ultraviolet light near a wavelength range of 260 nm - 270 nm having a high sterilizing efficiency. Such an ultraviolet LED is exemplified by an aluminum gallium nitride (AlGaN) based LED.

The sealing window 54 seals the light emitting device 52 and protects the light emitting device 52. The sealing window 54 is desirably made of a material having a high ultraviolet transmittance and is made of quartz (Si02), sapphire (A1203), amorphous fluororesin, or the like. For example, the sealing window 54 may have a bombshell shape as illustrated and, preferably, has a streamlined shape that does not block the flow of the fluid flowing out of the exit of the filter 40. The sealing window 54 may be shaped to have a lens function that adjusts light distribution property of the ultraviolet light from the light emitting device 52.

The reflector 56 reflects a portion of the ultraviolet light from the light emitting device 52 and adjusts the direction of the ultraviolet. The reflector 56 is provided to surround the light emitting device 52 and reflects the ultraviolet traveling from the light emitting device 52 toward the filter 40 in the direction of the downstream side of the second flow passage 33. The reflector 56 is made of a material having a high ultraviolet reflectivity such as aluminum and PTFE. The reflector 56 is sealed by the sealing window 54 along with the light emitting device 52.

With the above-described configuration, the fluid sterilization device 10 irradiates the fluid flowing in the second flow passage 33 with ultraviolet light so as to sterilize the fluid. The fluid flowing in via the inflow end 21 passes through the filter 40 made of hollow fibers 42. In other words, the fluid is filtered by the hollow fibers 42 by traveling from a space outside to a space inside and flows out of the openings at the ends 44. The filter 40 removes refuse such as fine particles included in the fluid, shapes the flow of the fluid in the first flow passage 23 by means of the plurality of hollow fibers 42 extending in the axial direction, turning the fluid into a laminar flow. The fluid flows out from the exit of the filter 40 in a laminar flow state and flows in the second flow passage 33, maintaining the laminar flow state. The light source 50 radiates ultraviolet light in the axial direction toward the fluid in the laminar flow state. The fluid irradiated with the ultraviolet light flows out via the outflow end 31.

According to the embodiment, the fluid turned into a laminar flow by the filter 40 extending in the longitudinal direction is irradiated with ultraviolet light so that the sterilization efficiency is increased as compared with the case of irradiating the fluid in a turbulent flow state with ultraviolet light. We have found that sterilization by ultraviolet irradiation in a straight flow passage is about 5-7 times more efficient in the case of a laminar flow state than in the case of a turbulent flow state. In the case of a turbulent flow, the fluid cannot be irradiated evenly with ultraviolet light so that a portion of the fluid flows out without receiving sufficient irradiation energy. Consequently, a portion of the fluid that is not sterilized sufficiently flows out, resulting in lower sterilization efficiency. Meanwhile, the embodiment causes the filter 40 to irradiate the fluid turned into a laminar flow with ultraviolet light and so ensures that the entire fluid is irradiated with a predefined amount of energy or higher and the sterilization effect is enhanced.

According to this embodiment, the light source 50 is positioned near the exit of the filter 40 to radiate ultraviolet light in a direction opposite to the filter 40. Therefore, the hollow fibers 42 and the sealant 46 forming the filter 40 are less likely to be irradiated with ultraviolet light than in the related art. Generally, the hollow fibers 42 are formed by a polymer resin such as polysulfone and polyether sulfone, and the sealant 46 is formed by a urethane resin, or the like. Both components are made of an organic material and so may be degraded by being irradiated with ultraviolet light. According to this embodiment, the device is configured such that irradiation of the hollow fibers 42 and the sealant 46 is inhibited so that the hollow fibers 42 and sealant 46 are prevented from being degraded and the durability and reliability of the fluid sterilization device 10 are improved.

According to this embodiment, the housing 12 is comprised of the first housing 20 and the second housing 30 and the first housing 20 and the second housing 30 are detachably connected so that maintenance of the interior of the housing 12 is easy. For example, where replacement of the filter 40 is required, the first housing 20 and the second housing 30 may be separated to remove the filter 40 to mount the new filter 40 easily. It is also easy to dismantle the housing 12 to clean the interior. Thus, according to this embodiment, the convenience of the fluid sterilization device 10 is improved.

### (Second embodiment)

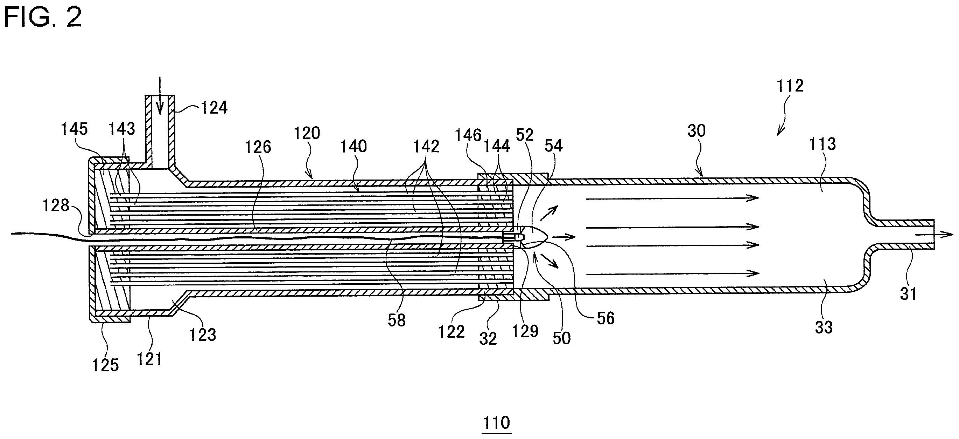

Fig. 2 is a cross sectional view schematically 25 showing a configuration of a fluid sterilization device 110 according to the second embodiment. The fluid sterilization device 110 differs from the first embodiment described above in that an inflow pipe 124 extending in a direction intersecting or perpendicular to the axial direction is provided at an inflow end 121 of a first housing 120. A description will be given of the fluid sterilization device 110, highlight differences from the first embodiment.

The fluid sterilization device 110 includes a housing 112 that defines a flow passage 113, a filter 140, and a light source 50. The housing 112 includes a first 10 housing 120 and a second housing 30.

The first housing 120 includes an inflow end 121, a first connection end 122, an inflow pipe 124, a cap 125, and a support structure 126. The first housing 120 extends from the inflow end 121 to the first connection end 122 in the longitudinal direction to define a first flow passage 123. The inflow end 121 is provided with the cap 125 that blocks the opening of the inflow end 121 and the inflow pipe 124 extending from the inflow end 121 in the radial direction. The fluid flowing in from the inflow pipe 124 in the axial direction changes its direction at the inflow end 121 and flows toward the first connection end 122 in the axial direction. The second connection end 32 of the second housing 30 is connected to the first connection end 122. The second housing 30 is configured similarly to that of the first embodiment.

The support structure 126 is provided near the central axis of the first housing 120. The support structure 126 extends in the axial direction from a wiring hole 128 provided near the center of the cap 125, and the light source 50 is mounted to an end 129 located in the first connection end 122. The wiring 58 is inserted in the support structure 126. The light source 50 is configured similarly to that of the first embodiment.

The filter 140 includes a plurality of hollow fibers 142, a first sealant 145, and a second sealant 146. The plurality of hollow fibers 142 are bundled such that first ends 143 thereof are aligned at the position of the inflow end 121, and second ends 144 are aligned at the position of the first connection end 122. The first ends 143 are fixed to the inflow end 121 and the cap 125 by means of the first sealant 145 and the openings thereof are blocked. The second ends 144 are fixed to the first connection end 122 by means of the second sealant 146 and the openings thereof are exposed at the end face on the downstream side.

According to the configuration above, the fluid sterilization device 110 filters the fluid flowing in from the inflow pipe 124 and creates a flow in a laminar flow state by means of the filter 40. The light source 50 radiates ultraviolet light in the direction of flow in the second flow passage 33 toward the fluid flowing from the exit of the filter 140 in a laminar flow state. The fluid sterilized by the ultraviolet light flows out from the outflow end 31. Accordingly, the same advantage as described above of the first embodiment is also obtained in this embodiment.

### (Third embodiment)

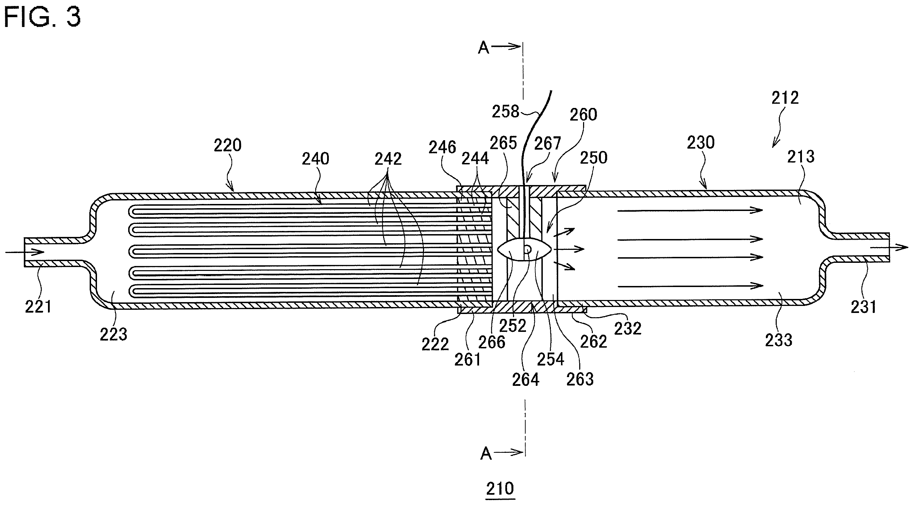

Fig. 3 is a cross sectional view schematically showing a configuration of a fluid sterilization device 210 according to the third embodiment. The fluid sterilization device 210 differs from the embodiments described above in that a housing 212 includes a first housing 220, a second housing 230, and a third housing 260, and a light source 250 is mounted to the third housing 260. A description will be given of the fluid sterilization device 210, highlight differences from the foregoing embodiments.

The fluid sterilization device 210 includes a housing 212 that defines a flow passage 213, a filter 240, and a light source 250. The housing 212 includes a first housing 220, a second housing 230, and a third housing 260.

The first housing 220 includes an inflow end 221 and a first connection end 222, and forms a first flow passage 223 extending from the inflow end 221 to the first connection end 222 in the longitudinal direction. The first connection end 222 is connected to a third connection end 261 of the third housing 260. The filter 240 is provided inside the first housing 220. The filter 240 includes a plurality of hollow fibers 242 and is configured similarly to the filter 40 according to the first embodiment. The plurality of hollow fibers 242 are provided to extend in the longitudinal direction and ends 244 thereof are fixed to the first connection end 222 by means of a sealant 246.

The second housing 230 includes an outflow end 231 and a second connection end 232 and forms a second flow passage 233 extending from the second connection end 232 to the outflow end 231 in the longitudinal direction. The second connection end 232 is connected to a fourth connection end 262 of the third housing 260.

The third housing 260 is connected between the first housing 220 and the second housing 230 and defines a third flow passage 263 connecting the first flow passage 223 and the second flow passage 233. The third housing 260 includes a third connection end 261, a fourth connection end 262, and a support structure 264. The support structure 264 includes a beam 265 extending from the side wall of the third housing 260 to the light source 250, and a base part 266 to which the light source 250 is mounted. A wiring hole 267 for a guiding a wiring 258 of the light source 250 is provided in the beam 265.

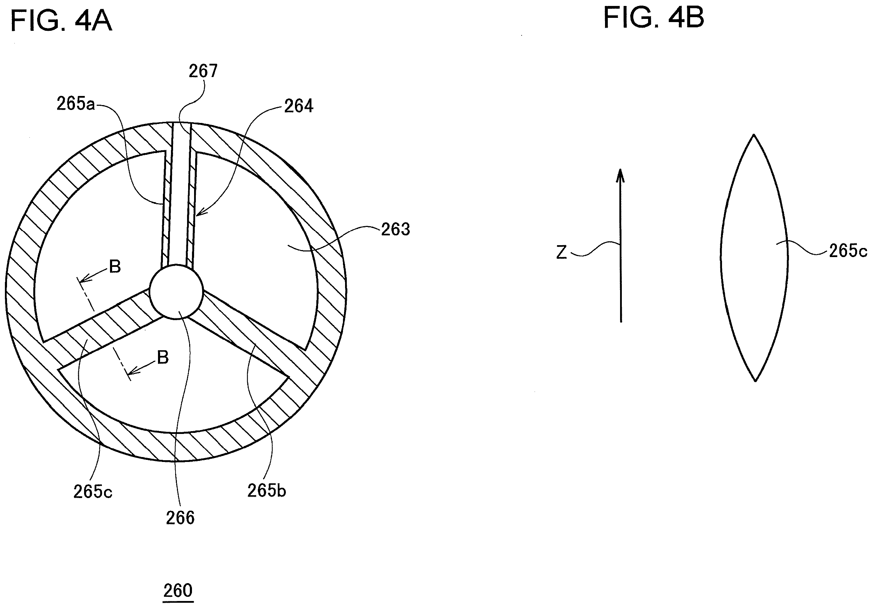

Figs. 4A and 4b are cross sectional views schematically showing a configuration of the support structure 264. Fig. 4A shows an A-A cross section of Fig. 3, and Fig. 4B shows a B-B cross section of Fig. 4A. The support structure 264 includes a first beam 265a, a second beam 265b, a third beam 265c, and the base part 266. The plurality of beams 265a-265c extend from respectively different positions on the side wall of the third housing 260 to the base part 266 located near the center of the third flow passage 263. The plurality of beams 265a-265c are shaped such that the cross section along the direction z of flow of the fluid is streamlined as shown in Fig. 4B. The base part 266 is shaped such that the cross section thereof along the axial direction is streamlined as shown in Fig. 3.

The light source 250 includes a light emitting device 252 emitting ultraviolet light and a sealing window 254. The light source 250 is mounted to the base part 266 and positioned near the central axis of the third flow passage 263. The light source 250 radiates ultraviolet light in the axial direction toward the fluid flowing in the second flow passage 233.

With the above-described configuration, the fluid sterilization device 210 irradiates the fluid which passes through the first flow passage 223 and is turned into a laminar flow by the filter 240 to flow in the second flow passage 233, with ultraviolet light so as to sterilize the fluid. The streamlined shape of the support structure 264 for fixing the light source 250 does not block the flow of the fluid turned into a laminar flow by the filter 240 and allows the fluid to flow in the second flow passage 233, maintaining the laminar flow state. Accordingly, the same advantage as described above of the foregoing embodiments is also obtained in this embodiment.

According to this embodiment, the light source 250 is not provided in the first housing 220 and is provided in the third housing 260 that can be detached from the first housing 220. Therefore, the structure of the first housing 220 including the filter 240 is simplified. Further, the maintenance job to replace the filter 240 is more simplified. The user of the fluid sterilization device 210 can replace the filter 240 by removing the first housing 220 from the third housing 260 or replace the filter 240 by exchanging the entirety of the first housing 220 including the filter 240. In this way, the convenience of the fluid sterilization device 210 is improved.

### (Fourth embodiment)

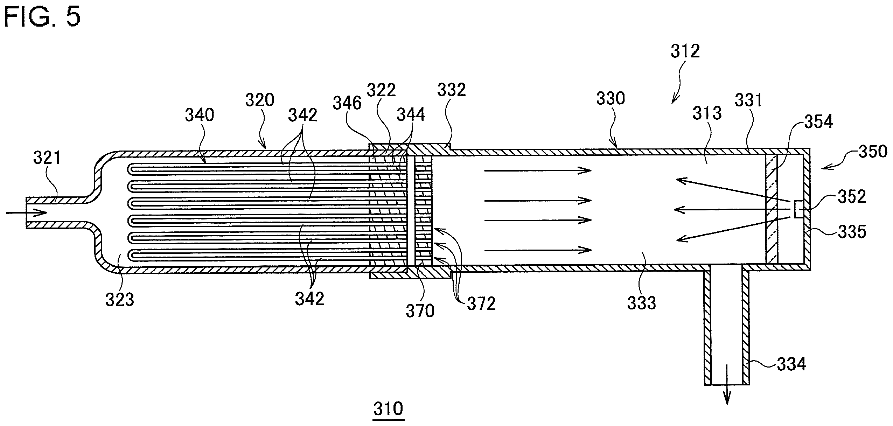

Fig. 5 is a cross sectional view schematically showing a configuration of a fluid sterilization device 310 according to the fourth embodiment. The fluid sterilization device 310 differs from the embodiments described above in that a light source 350 is provided at an outflow end 331 of a second housing 330. A description will be given of the fluid sterilization device 310, highlight differences from the foregoing embodiments.

The fluid sterilization device 310 includes a housing 312 that defines a flow passage 313, a filter 340, a light source 350, and a straightener 370. The housing 312 includes a first housing 320 and a second housing 330.

The first housing 320 is configured similarly to the first housing 220 described above. The first housing 320 includes an inflow end 321 and a first connection end 322 and forms a first flow passage 323 extending from the inflow end 321 to the first connection end 322 in the longitudinal direction. The filter 340 is provided inside the first housing 320. The filter 340 has a plurality of hollow fibers 342 extending in the axial direction. Ends 344 of the plurality of hollow fibers 342 are fixed to the first connection end 322 by means of a sealant 346.

The second housing 330 includes an outflow end 331, a second connection end 332, an outflow pipe 334, and an end face wall 335. The second housing 330 extends from the second connection end 332 to the outflow end 331 in the longitudinal direction to define a second flow passage 333. The outflow end 331 is provided with the end face wall 335 perpendicular to the axial direction of the second flow passage 333 and the outflow pipe 334 extending from the outflow end 331 in the radial direction. The fluid flowing in the second flow passage 333 in the axial direction changes its direction at the outflow end 331 and flows out in the radial direction via the outflow pipe 334.

The light source 350 includes a light emitting device 352 and a sealing window 354 and is provided at the outflow end 331 at the end of the flow passage 313. The light emitting device 352 is mounted near the center of the end face wall 335 and emits ultraviolet light toward the second flow passage 333. The sealing window 354 is a plate member isolating the light emitting device 352 and the second flow passage 333 and is made of quartz (SiO2), sapphire (A1203), amorphous fluororesin, or the like. The sealing window 354 transmits the ultraviolet light from the light emitting device 352 and prevents the fluid flowing in the second flow passage 333 from coming into contact with the light emitting device 352.

The straightener 370 is provided at the second connection end 332. The straightener 370 has a plurality of water pores 372 to straighten the flow of the fluid flowing out from the exit of the filter⁻340 into a laminar flow. The straightener 370 is made of a material that reflects ultraviolet light and is made of, for example, aluminum or PTFE. The straightener 370 reflects the ultraviolet light from the light source 350 and guides it toward the downstream side of the second flow passage 333, and shields the ultraviolet light to prevent the ultraviolet light from the light source 350 from irradiating the hollow fibers 342 and the sealant 346 forming the filter 340. The straightener 370 is preferably configured such that the thickness of the straightener 370 in the axial direction is larger than the diameter of the plurality of water pores 372 so as to prevent the ultraviolet light from leaking via the plurality of water pores 372.

With the above-described configuration, the fluid sterilization device 310 irradiates the fluid flowing in the second flow passage 333 with ultraviolet light so as to sterilize the fluid. The fluid flowing in from the inflow end 321 is turned into a laminar flow by the filter 340 and the straightener 370, and the fluid flowing in the second flow passage 333 in a laminar flow state is irradiated with ultraviolet light. The light source 350 radiates ultraviolet light in the direction of flow of the fluid in the second flow passage 333. The same advantage as described above of the foregoing embodiments is also obtained in this embodiment.

According to this embodiment, the light source 350 is provided in the second housing 330 instead of the first housing 320. Therefore, it is easy to replace the filter 340 as in the case of the third embodiment described above. By providing the straightener 370 near the exit of the filter 340, the ultraviolet light traveling from the light source 350 to the filter 340 is shielded so as to protect the filter 340 from the ultraviolet light. This improves the durability and reliability of the fluid sterilization device 310.

### (Fifth embodiment)

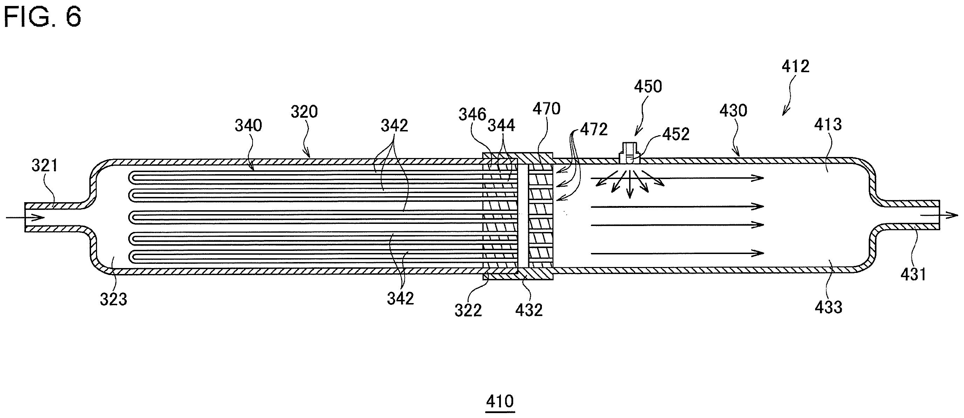

Fig. 6 is a cross sectional view schematically showing a configuration of a fluid sterilization device 410 according to the fifth embodiment. The fluid sterilization device 410 differs from the embodiments described above in that a light source 450 is provided in the side wall of a second housing 430. A description will be given of the fluid sterilization device 410, highlight differences from the foregoing embodiments.

The fluid sterilization device 410 includes a housing 412 that defines a flow passage 413, a filter 340, and a light source 450. The housing 412 includes a first housing 320 and a second housing 430. The first housing 320 and the filter 340 are configured similarly to that of the fourth embodiment.

The second housing 430 defines a second flow passage 433 extending in a longitudinal direction from a second connection end 432 to an outflow end 431. The side wall of the second housing 430 is provided with the light source 450.
The light source 450 includes a light emitting device 452 that emits ultraviolet light and radiates ultraviolet light in the direction intersecting the axial direction toward the fluid flowing in the second flow passage 433. A straightener 470 is provided at the second connection end 432. The straightener 470 has a plurality of water pores 472 to straighten the flow of the fluid flowing out from the exit of the filter 340 into a laminar flow. Further, the straightener 470 shields the ultraviolet light from the light source 450 so as to protect the filter 340.

With the above-described configuration, the fluid sterilization device 410 irradiates the fluid flowing in the second flow passage 433 with ultraviolet light so as to sterilize the fluid. The fluid flowing in from the inflow end 321 is turned into a laminar flow by the filter 340 and the straightener 470 and the fluid flowing in the second flow passage 433 in a laminar flow state is irradiated with ultraviolet light. The light source 450 radiates ultraviolet light in the direction intersecting the flow of the fluid in the second flow passage 433. The ultraviolet light traveling from the light source 450 to the filter 340 is shielded by the straightener 470. The same advantage as described above of the foregoing embodiments is also obtained in this embodiment.

Described above is an explanation based on an exemplary embodiment. The embodiment is intended to be illustrative only and it will be obvious to those skilled in the art that various modifications to constituting elements and processes could be developed and that such modifications are also within the scope of the present invention.

The fluid sterilization device according to the embodiments described above is described as an device for irradiating the fluid with ultraviolet light so as to sterilize the fluid. In one variation, the inventive sterilization device may be used for a purification process that decomposes organic substance included in a fluid by using ultraviolet irradiation.

In the embodiments described above, hollow fibers are described as being used in the filter. In one variation, a filter in which activated carbon is used or a special filter that selectively adsorbs hazardous substance such as arsenic may be used. Alternatively, a hollow fiber filter and any of those filters may be used in combination.

In one variation, a hydraulic power generator unit may be provided inside the housing and the light source may be turned on by using the electric power generated by using the flow of the fluid.

### [DESCRIPTION OF THE REFERENCE NUMERALS]

10 ... fluid sterilization device, 12 ... housing, 13 ... flow passage, 26 ... support structure, 40 ... filter, 50 ... light source, 52 ... light emitting device, 110 . fluid sterilization device, 112 ... housing, 113 ... flow passage, 126 ... support structure, 140 ....filter, 210 ... fluid sterilization device, 212 ... housing, 213 ... flow passage, 240 ... filter, 250 ... light source, 252 light emitting device, 264 ... support structure, 310 ... fluid sterilization device, 312 .... housing, 313 ... flow passage, 340 ... filter, 350 ... light source, 352 ... light emitting device, 370 ... straightener, 410 ... fluid sterilization device, 412 ... housing, 413 ... flow passage, 450 ... light source, 452 ... light emitting device, 470 ... straightener

### [INDUSTRIAL APPLICABILITY]

According to the present invention, the efficiency of irradiating the fluid flowing in the flow passage with ultraviolet light is increased and the sterilization capability is improved.

## Claims

1. A fluid sterilization device (10, 110, 210) comprising:
a housing (12, 112, 212) including a first housing (20, 120, 220) that defines an upstream side of a flow passage (13, 113, 213) extending in a longitudinal direction and a second housing (30, 230) that defines a downstream side of the flow passage and that is connectable to the first housing;
a hollow fiber filter (42, 142, 242) provided in the first housing and extending in the longitudinal direction; and
a light source (50, 250) provided in the housing and including a light emitting diode (52, 252) that emits ultraviolet light, a reflector (56) that is provided to surround the light emitting diode, and a sealing window (54, 254) that seals the light emitting diode and the reflector, wherein the light source is positioned near an exit of the hollow fiber filter on the central axis of the housing to radiate the ultraviolet light toward the downstream side of the flow passage opposite to the hollow fiber filter, and wherein the reflector reflects the ultraviolet light traveling from the light emitting diode toward the filter in the direction of the downstream side of the flow passage so as to irradiate a fluid flowing in a laminar flow state in the second housing after passing through the hollow fiber filter with the ultraviolet light.

2. The fluid sterilization device (10, 110, 210) according to claim 1, wherein
the sealing window has a streamlined shape extending towards the downstream side of the housing.

3. The fluid sterilization device (10, 110) according to claim 1 or 2, wherein
the second housing is threadably and detachably connectable to the first housing, and
the light source is positioned near a boundary between the first housing and the second housing.

4. The fluid sterilization device (10, 110) according to any one of claims 1 to 3, further comprising:
a support structure (26, 126) for fixing the light source near a central axis of the flow passage, wherein
the support structure is a pipe member that extends along the hollow fiber filter in the longitudinal direction near the central axis of the flow passage.

5. The fluid sterilization device (210) according to any one of claims 1 to 3, further comprising:
a third housing (260) that is connectable between the first housing and the second housing; and
a support structure (264) for fixing the light source near a central axis of the flow passage, wherein
the support structure (264) has a beam (265a, 265b, 265c) extending in a radial direction from a side wall of the third housing to the central axis of the flow passage and a base (266) to which the light source is mounted near the central axis of the flow passage, and
the beam and the base have a streamlined cross section along the longitudinal direction.

6. The fluid sterilization device (10, 110, 210) according to claim 4 or 5, wherein
a wiring (58, 258) for supplying electric power to the light source (50, 250) is provided inside the support structure (26, 126, 264).

7. A fluid sterilization device (310, 410) comprising:
a housing (312, 412) including a first housing (320) that defines an upstream side of a flow passage (313, 413) extending in a longitudinal direction and a second housing (330, 430) that defines a downstream side of the flow passage and that is connectable to the first housing;
a hollow fiber filter (340) provided in the first housing and extending in the longitudinal direction;
a straightener (370, 470) is provided near an exit of the hollow fiber filter (340), wherein the straightener has a plurality of water pores (372) to pass a fluid after passing through the hollow fiber filter (340), and wherein the straightener is made of aluminum or PTFE; and
a light source (350, 450) including a light emitting diode (352, 452) that emits ultraviolet light and a sealing window (354), wherein the light source is provided near the center of the end face wall (335) of the second housing and irradiates a fluid flowing in a laminar flow state in a downstream side of the housing after passing through the straightener (370,470) with the ultraviolet light, wherein
the straightener (370,470) is configured such that a thickness of the straightener in the longitudinal direction is larger than a diameter of the plurality of water pores so as to shield the ultraviolet light traveling toward the hollow fiber filter (340).

8. The fluid sterilization device (310) according to claim 7, wherein
the light source (350) is provided at the downstream end of the second housing and radiates the ultraviolet light in the longitudinal direction toward the upstream side of the flow passage.

9. The fluid sterilization device (410) according to claim 7, wherein
the light source (450) is provided at the side wall of the second housing (430) and radiates the ultraviolet light in a direction intersecting the longitudinal direction.

## Patentansprüche

1. Fluidsterilisationsvorrichtung (10, 110, 210), Folgendes umfassend:
ein Gehäuse (12, 112, 212), das ein erstes Gehäuse (20, 120, 220), das eine stromaufwärtige Seite eines Strömungsdurchgangs (13, 113, 213) definiert, der sich in eine Längsrichtung erstreckt, und ein zweites Gehäuse (30, 230) umfasst, das eine stromabwärtige Seite des Strömungsdurchgangs definiert und das an das erste Gehäuse anschließbar ist;
einen Hohlfaserfilter (42, 142, 242), der in dem ersten Gehäuse bereitgestellt ist und sich in die Längsrichtung erstreckt; und
eine Lichtquelle (50, 250), die in dem Gehäuse bereitgestellt ist und eine Leuchtdiode (52, 252), die Ultraviolettlicht emittiert, einen Reflektor (56), der bereitgestellt ist, um die Leuchtdiode zu umgeben, und ein Dichtungsfenster (54, 254) umfasst, das die Leuchtdiode und den Reflektor abdichtet, wobei die Lichtquelle nahe einem Ausgang des Hohlfaserfilters auf der zentralen Achse des Gehäuses positioniert ist, um das Ultraviolettlicht in Richtung der stromabwärtigen Seite des Strömungsdurchgangs entgegengesetzt zu dem Hohlfaserfilter abzustrahlen, und wobei der Reflektor das Ultraviolettlicht reflektiert, das aus der Leuchtdiode in Richtung des Filters in die Richtung der stromabwärtigen Seite des Strömungsdurchgangs strahlt, um so ein Fluid, das in einem laminaren Strömungszustand in dem zweiten Gehäuse strömt, nach Passieren durch den Hohlfaserfilter mit dem Ultraviolettlicht zu bestrahlen.

2. Fluidsterilisationsvorrichtung (10, 110, 210) nach Anspruch 1, wobei
das Dichtungsfenster eine stromlinienförmige Gestalt aufweist, die sich in Richtung der stromabwärtigen Seite des Gehäuses erstreckt.

3. Fluidsterilisationsvorrichtung (10, 110) nach Anspruch 1 oder 2, wobei
das zweite Gehäuse anschraubbar und abnehmbar an das erste Gehäuse anschließbar ist, und
die Lichtquelle nahe einer Grenze zwischen dem ersten Gehäuse und dem zweiten Gehäuse positioniert ist.

4. Fluidsterilisationsvorrichtung (10, 110) nach einem der Ansprüche 1 bis 3, weiterhin Folgendes umfassend:
eine Tragestruktur (26, 126) zum Befestigen der Lichtquelle nahe einer zentralen Achse des Strömungsdurchgangs, wobei
die Tragestruktur ein Rohrglied ist, das sich entlang des Hohlfaserfilters in die Längsrichtung nahe der zentralen Achse des Strömungsdurchgangs erstreckt.

5. Fluidsterilisationsvorrichtung (210) nach einem der Ansprüche 1 bis 3, weiterhin Folgendes umfassend:
ein drittes Gehäuse (260), das zwischen dem ersten Gehäuse und dem zweiten Gehäuse anschließbar ist; und
eine Tragestruktur (264) zum Befestigen der Lichtquelle nahe einer zentralen Achse des Strömungsdurchgangs, wobei die Tragestruktur (264) einen Träger (265a, 265b, 265c), der sich in eine radiale Richtung von einer Seitenwand des dritten Gehäuses zu der zentralen Achse des Strömungsdurchgangs erstreckt, und eine Basis (266) aufweist, an der die Lichtquelle nahe der zentralen Achse des Strömungsdurchgangs befestigt ist, und
der Träger und die Basis einen stromlinienförmigen Querschnitt entlang der Längsrichtung aufweisen.

6. Fluidsterilisationsvorrichtung (10, 110, 210) nach Anspruch 4 oder 5, wobei
eine Verdrahtung (58, 258) zum Liefern von elektrischem Strom an die Lichtquelle (50, 250) innerhalb der Tragestruktur (26, 126, 264) bereitgestellt ist.

7. Fluidsterilisationsvorrichtung (310, 410), Folgendes umfassend:
ein Gehäuse (312, 412), das ein erstes Gehäuse (320), das eine stromaufwärtige Seite eines Strömungsdurchgangs (313, 413) definiert, der sich in eine Längsrichtung erstreckt, und ein zweites Gehäuse (330, 430) umfasst, das eine stromabwärtige Seite des Strömungsdurchgangs definiert und das an das erste Gehäuse anschließbar ist; einen Hohlfaserfilter (340), der in dem ersten Gehäuse bereitgestellt ist und sich in die Längsrichtung erstreckt; und
einen Richtapparat (370, 470), der nahe einem Ausgang des Hohlfaserfilters (340) bereitgestellt ist, wobei der Richtapparat mehrere Wasserporen (372) aufweist, um ein Fluid nach Passieren durch den Hohlfaserfilter (340) durchzulassen, und wobei der Richtapparat aus Aluminium oder PTFE angefertigt ist; und
eine Lichtquelle (350, 450), die eine Leuchtdiode (352, 452), die Ultraviolettlicht emittiert. und ein Dichtungsfenster (354) umfasst, wobei die Lichtquelle nahe der Mitte der Endflächenwand (335) des zweiten Gehäuses bereitgestellt ist und ein Fluid, das in einem laminaren Strömungszustand in einer stromabwärtigen Seite des Gehäuses strömt, nach Passieren durch den Richtapparat (370, 470) mit dem Ultraviolettlicht bestrahlt, wobei
der Richtapparat (370, 470) derartig eingerichtet ist, dass eine Dicke des Richtapparats in der Längsrichtung größer ist als ein Durchmesser der mehreren Wasserporen, um so das Ultraviolettlicht abzuschirmen, das in Richtung des Hohlfaserfilters (340) strahlt.

8. Fluidsterilisationsvorrichtung (310) nach Anspruch 7, wobei
die Lichtquelle (350) an dem stromabwärtigen Ende des zweiten Gehäuses bereitgestellt ist und das Ultraviolettlicht in die Längsrichtung in Richtung der stromaufwärtigen Seite des Strömungsdurchgangs strahlt.

9. Fluidsterilisationsvorrichtung (410) nach Anspruch 7, wobei
die Lichtquelle (450) an der Seitenwand des zweiten Gehäuses (430) bereitgestellt ist und das Ultraviolettlicht in eine Richtung strahlt, welche die Längsrichtung kreuzt.

## Revendications

1. Dispositif de stérilisation de fluide (10, 110, 210) comprenant :
un boîtier (12, 112, 212) comprenant un premier boîtier (20, 120, 220) qui définit un côté amont d'un passage d'écoulement (13, 113, 213) s'étendant dans une direction longitudinale et un deuxième boîtier (30, 230) qui définit un côté aval du passage d'écoulement et qui peut être raccordé au premier boîtier ;
un filtre à fibres creuses (42, 142, 242) disposé dans le premier boîtier et s'étendant dans la direction longitudinale ; et
une source de lumière (50, 250) placée dans le boîtier et comprenant une diode électroluminescente (52, 252) qui émet une lumière ultraviolette, un réflecteur (56) qui est fourni pour entourer la diode électroluminescente, et une fenêtre d'étanchéité (54, 254) qui assure l'étanchéité de la diode électroluminescente et du réflecteur, la source de lumière étant positionnée près d'une sortie du filtre à fibres creuses sur l'axe central du boîtier pour diffuser la lumière ultraviolette en direction du côté aval du passage d'écoulement à l'opposé du filtre à fibres creuses, et le réflecteur réfléchissant la lumière ultraviolette se déplaçant depuis la diode électroluminescente en direction du filtre dans la direction du côté aval du passage d'écoulement de façon à exposer à la lumière ultraviolette un fluide s'écoulant dans un état de flux laminaire dans le deuxième boîtier après être passé à travers le filtre à fibres creuses.

2. Dispositif de stérilisation de fluide (10, 110, 210) selon la revendication 1, dans lequel
la fenêtre d'étanchéité a une forme profilée s'étendant en direction du côté aval du boîtier.

3. Dispositif de stérilisation de fluide (10, 110) selon la revendication 1 ou 2, dans lequel le deuxième boîtier peut être relié par vissage et de façon détachable au premier boîtier, et
la source de lumière est positionnée près d'une frontière entre le premier boîtier et le deuxième boîtier.

4. Dispositif de stérilisation de fluide (10, 110) selon l'une quelconque des revendications 1 à 3, comprenant en outre :
une structure de support (26, 126) destinée à fixer la source de lumière près d'un axe central du passage d'écoulement, dans lequel
la structure de support est un élément de tuyau qui s'étend le long du filtre à fibres creuses dans la direction longitudinale près de l'axe central du passage d'écoulement.

5. Dispositif de stérilisation de fluide (210) selon l'une quelconque des revendications 1 à 3, comprenant en outre :
un troisième boîtier (260) qui peut être relié entre le premier boîtier et le deuxième boîtier ; et
une structure de support (264) destinée à fixer la source de lumière près d'un axe central du passage d'écoulement, dans lequel
la structure support (264) possède un faisceau (265a, 265b, 265c) s'étendant dans une direction radiale depuis une paroi latérale du troisième boîtier vers l'axe central du passage d'écoulement et une base (266) sur laquelle est montée la source de lumière près de l'axe central du passage d'écoulement, et
le faisceau et la base ont une coupe transversale profilée le long de la direction longitudinale.

6. Dispositif de stérilisation de fluide (10, 110, 210) selon la revendication 4 ou 5, dans lequel un câblage (58, 258) destiné à fournir de l'énergie électrique à la source de lumière (50, 250) est prévu à l'intérieur de la structure de support (26, 126, 264).

7. Dispositif de stérilisation de fluide (310, 410) comprenant :
un boîtier (312, 412) comprenant un premier boîtier (320) qui définit un côté amont d'un passage d'écoulement (313, 413) s'étendant dans une direction longitudinale et un deuxième boîtier (330, 430) qui définit un côté aval du passage d'écoulement et qui peut être raccordé au premier boîtier ;
un filtre à fibres creuses (340) disposé dans le premier boîtier et s'étendant dans la direction longitudinale ; un redresseur (370, 470) est prévu près d'une sortie du filtre à fibres creuses (340), le redresseur comportant une pluralité de pores (372) pour l'eau pour faire passer un fluide après un passage à travers le filtre à fibres creuses (340), et dans lequel le redresseur est en aluminium ou PTFE ; et
une source de lumière (350, 450) comprenant une diode électroluminescente (352, 452) qui émet une lumière ultraviolette et une fenêtre d'étanchéité (354), la source de lumière étant positionnée près du centre de la paroi (335) de face d'extrémité du deuxième boîtier et exposant à la lumière ultraviolette un fluide s'écoulant dans un état de flux laminaire dans un côté aval du boîtier après être passé à travers le raidisseur (370, 470), dans lequel
le redresseur (370, 470) est conçu de sorte qu'une épaisseur du redresseur dans la direction longitudinale soit plus grande qu'un diamètre de la pluralité de pores pour l'eau de façon à protéger la lumière ultraviolette se déplaçant en direction du filtre à fibres creuses (340) .

8. Dispositif de stérilisation de fluide (310) selon la revendication 7, dans lequel
la source de lumière (350) est prévue au niveau de l'extrémité aval du deuxième boîtier et diffuse la lumière ultraviolette dans la direction longitudinale en direction du côté amont du passage d'écoulement.

9. Dispositif de stérilisation de fluide (410) selon la revendication 7, dans lequel
la source de lumière (450) est prévue au niveau de la paroi latérale du deuxième boîtier (430) et diffuse la lumière ultraviolette dans une direction coupant la direction longitudinale.
